(19) Europäisches Patentamt
European Patent Office
Office européen des brevets

(11) Veröffentlichungsnummer: **0 078 999**
**A1**

(12) # EUROPÄISCHE PATENTANMELDUNG

(21) Anmeldenummer: 82109970.2

(22) Anmeldetag: 28.10.82

(51) Int. Cl.³: C 07 D 261/04
C 07 D 413/12, A 01 N 43/80

(30) Priorität: 06.11.81 DE 3144140

(43) Veröffentlichungstag der Anmeldung: 18.05.83 Patentblatt 83/20

(84) Benannte Vertragsstaaten: AT BE CH DE FR GB IT LI NL SE

(71) Anmelder: BASF Aktiengesellschaft
Carl-Bosch-Strasse 38
D-6700 Ludwigshafen(DE)

(72) Erfinder: Meyer, Norbert, Dr.
Stahlbuehlring 155A
D-6802 Ladenburg(DE)

(72) Erfinder: Zeeh, Bernd, Dr.
Thorwaldsenstrasse 5
D-6700 Ludwigshafen(DE)

(72) Erfinder: Rentzea, Costin, Dr.
Neuenheimer Landstrasse 72
D-6900 Heidelberg(DE)

(72) Erfinder: Pommer, Ernst-Heinrich, Dr.
Berliner Platz 7
D-6703 Limburgerhof(DE)

(72) Erfinder: Ammermann, Eberhard, Dr.
Sachsenstrasse 3
D-6700 Ludwigshafen(DE)

(54) **2-Isoxazolincarbonsäureamide, Verfahren zu ihrer Herstellung und diese enthaltende Fungizide.**

(57) 2-Isoxazolincarbonsäureamide der allgemeinen Formel I

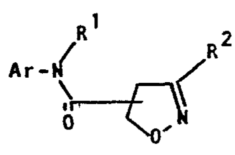

I

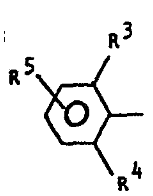

oder

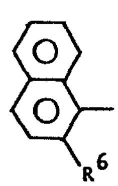

bedeutet,

und
$R^3$ Alkyl oder Halogen, $R^4$ Alkyl, $R^5$ Wasserstoff, Alkyl oder Alkoxy, $R^6$ Methyl und

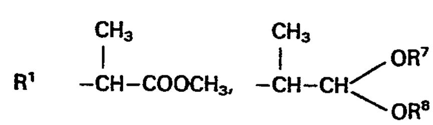

oder

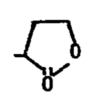

bedeutet,
wobei
$R^7$ und $R^8$ Methyl Ethyl bedeuten oder $R^7$ und $R^8$ zusammen eine Alkylengruppe bedeuten, und $R^2$ Wasserstoff, Alkyl oder einen gegebenenfalls substituierten Phenylrest bedeutet und diese enthaltende Fungizide.

EP 0 078 999 A1

Croydon Printing Company Ltd.

# 2-Isoxazolincarbonsäureamide, Verfahren zu ihrer Herstellung und diese enthaltende Fungizide

Die vorliegende Erfindung betrifft neue wertvolle 2-Isoxazolincarbonsäureamide, Verfahren zu ihrer Herstellung und Fungizide, die diese Verbindungen enthalten.

Es ist bereits bekannt, das Zink-ethylen-1,2-bisdithiocarbamat, das N-Trichlormethylthiophthalimid oder das Trichlormethylthiotetrahydro-phthalimid als Fungizide zu verwenden (vgl. R. Wegler, "Chemie der Pflanzenschutz- und Schädlingsbekämpfungsmittel", Band 2, Seiten 65, 66 und 109 und Band 4, Seiten 139 und 191,Berlin/Heidelberg/New York (1970) und (1977)).

Jedoch sind diese Fungizide nicht nach erfolgter Infektion verwendbar und ihre Wirkung bei niedrigen Aufwandkonzentrationen genügt nicht den Anforderungen der Praxis.

Es wurde nun gefunden, daß neue 2-Isoxazolincarbonsäureamide der allgemeinen Formel I

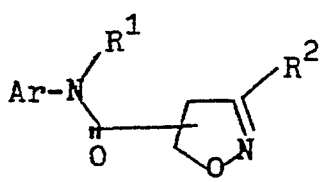

I

worin Ar

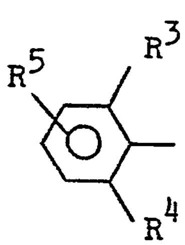 oder 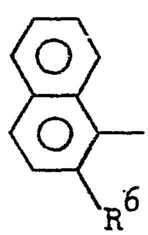 bedeutet,

Sws/P

$R^3$ $C_1$-$C_3$-Alkyl oder Halogen, $R^4$ $C_1$-$C_3$-Alkyl, $R^5$ Wasserstoff, $C_1$-$C_3$-Alkyl oder $C_1$-$C_3$-Alkoxy, $R^6$ Methyl und

$R^1$ $-\overset{CH_3}{\overset{|}{CH}}-COOCH_3$, $-\overset{CH_3}{\overset{|}{CH}}-CH\begin{smallmatrix}OR^7\\OR^8\end{smallmatrix}$ oder 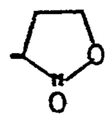 bedeutet, wobei

$R^7$ und $R^8$ Methyl oder Ethyl bedeuten oder $R^7$ und $R^8$ zusammen eine $C_2$-$C_3$-Alkylengruppe bedeuten, die zusammen mit dem Rest $-CH\begin{smallmatrix}O-\\O-\end{smallmatrix}$ einen gegebenenfalls durch $C_1$-$C_3$-Alkyl substituierten Dioxolan- oder 1,3-Dioxanring bilden und

$R^2$ Wasserstoff, $C_1$-$C_4$-Alkyl oder einen ggf. durch Halogen substituierten Phenylrest bedeutet, eine gute fungizide Wirkung haben.

$R^3$ bedeutet beispielsweise Methyl, Ethyl, Propyl, Isopropyl, Fluor, Chlor, Brom

$R^4$ bedeutet beispielsweise Methyl, Ethyl, Propyl, Isopropyl

$R^5$ bedeutet beispielsweise Wasserstoff, Methyl, Ethyl, Propyl, Isopropyl, Methoxy, Ethoxy, Propoxy, Isopropoxy.

$R^2$ bedeutet beispielsweise Wasserstoff, Methyl, Ethyl, Propyl, Isopropyl, Butyl, tert.-Butyl, Phenyl, 4--Chlorphenyl, 3,4-Dichlorphenyl, 4-Bromphenyl, 4--Fluorphenyl.

Bevorzugt werden die folgenden Bedeutungen: $R^2$ Methyl, $R^3$ und $R^4$ Methyl, Ethyl und $R^5$ Wasserstoff.

Die neuen 2-Isoxazolincarbonsäureamide der Formel I besitzen chirale Kohlenstoffatome im Rest $R^1$ und im Isoxazolinring sowie je nach der Beschaffenheit von $R^1$ noch weitere Chiralitätszentren. Nach üblichen Methoden können die

optisch reinen Enantiomeren bzw. die Diastereomeren erhalten werden. Die vorliegende Erfindung erfaßt auch diese Verbindungen in reiner Form oder als Mischungen. Als Fungizide sind sowohl die reinen Enantiomeren bzw. die einheitlichen Diasteromeren als auch die bei der Synthese üblicherweise anfallenden Mischungen wirksam.

Weiterhin wurde gefunden, daß man die neuen Verbindungen der allgemeinen Formel (I) erhält, wenn man ein aromatisches Amin der Formel II

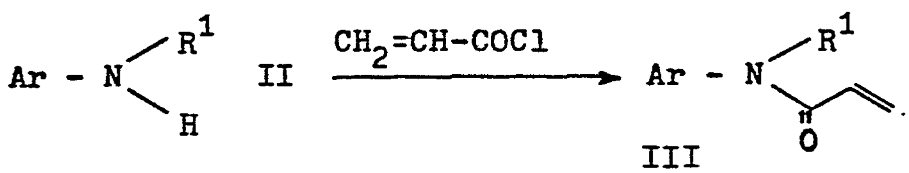


worin
Ar und $R^1$ die oben angegebenen Bedeutungen haben mit Acrylsäurechlorid gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers, bei Temperaturen zwischen -10 und +100°C zu einem Acrylsäureamid der Formel III umsetzt. Als bevorzugte, gegenüber den Reaktionsteilnehmern inerte Lösungs- oder Verdünnungsmittel werden z.B. aliphatische oder aromatische Kohlenwasserstoffe, wie Pentan, Cyclohexan, Petrolether, Benzol, Toluol oder Xylole; Halogenkohlenwasserstoffe, beispielsweise Methylenchlorid, Chloroform, 1,2-Dichlorethan, Chlorbenzol; Ketone wie Aceton und Methylethylketon; Ether wie Diethylether, Dimethoxyethan, Tetrahydrofuran oder Dioxan; Ester wie Essigsäureethylester; Nitrile wie Acetonitril; Sulfoxide wie Dimethylsulfoxid oder entsprechende Gemische verwendet.

Geeignete anorganische oder organische Basen, die gegebenenfalls auch als säurebindende Mittel bei der Reaktion

verwendet werden können, sind beispielsweise Alkali- und Erdalkalicarbonate wie Natrium- oder Kaliumbicarbonat, Natrium- oder Kaliumcarbonat, Calciumcarbonat; Borate wie Natriumborat; Phosphate wie Natrium- oder Kaliumdi- oder -triphosphat oder Amine wie Triethylamin, N,N-Dimethylanilin, N,N-Dimethylcyclohexylamin, N-Methylpiperidin oder Pyridin. Es können aber auch andere übliche Basen verwendet werden.

Als Reaktionsbeschleuniger kommen vorzugsweise Metallhalogenide wie Natriumbromid oder Kaliumjodid, Azole wie Imidazol oder 1,2,4-Triazol, Pyridine wie 4-Dimethylaminopyridin oder Dimethylformamid in Frage.

Die erfindungsgemäßen Umsetzungen erfolgen beispielsweise bei Temperaturen zwischen -10 und +100°C, vorzugsweise zwischen 0 und +40°C, drucklos oder unter Druck, kontinuierlich oder diskontinuierlich.

Die Umsetzung der Acrylsäureamide der allgemeinen Formel III zu den 2-Isoxazolincarbonsäureamiden der Formel I erfolgt durch Cycloaddition eines Nitriloxids der allgemeinen Formel IV

$$R^2-C\equiv\overset{\oplus}{N}-O^{\ominus} \qquad IV,$$

in der $R^2$ die oben genannten Bedeutungen hat, in Gegenwart eines Lösungsmittels bei einer Temperatur zwischen -10°C und +60°C.

Verfahren zur Herstellung und Umsetzung von Nitriloxiden sind bekannt (z.B. Houben-Weyl-Müller, Methoden der organischen Chemie, Band X/3, Seiten 843ff, 858ff, Stuttgart 1965). Bevorzugt werden zur Synthese der 2-Isoxazolincarbonsäureamide Oxime nach DE-OS 2 754 832 zu Nitriloxden

oxidiert und in situ an die Acrylsäureamide der Formel IV addiert.

Als Oxidationsmittel verwendet man z.B. handelsübliche wäßrige Hypochloritlösungen; geeignete Lösungsmittel sind z.B. Wasser, Alkohole wie Methanol, Ethanol, Propanol, Ketone wie Aceton, Methylethylketon, Ether wie Diethylether, Tetrahydrofuran, chlorierte aliphatische Kohlenwasserstoffe wie Methylenchlorid, Chloroform, Tetrachlorethan, aromatische Verbindungen wie Benzol, Toluol, Xylole, Chlorbenzole, Essigester, Dimethylformamid, Dimethylsulfoxid.

Die Cycloaddition wird bei Temperaturen im Bereich von -10 bis +60°C durchgeführt.

Die Ausgangsstoffe der allgemeinen Formel II sind teilweise bekannt; sie werden nach an sich allgemein bekannten Methoden hergestellt.

Die Herstellung der neuen Verbindungen wird durch folgende Beispiele erläutert:

Beispiel 1

Herstellung der Verbindung

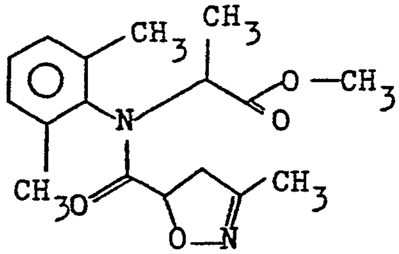

N-(3-Methyl-2-isoxazolin-5-yl-carbonyl)-N-(2,6-dimethylphenyl)-2-aminopropionsäuremethylester

a) Herstellung von N-Acryloyl-N-(2,6-dimethylphenyl)--2-aminopropionsäuremethylester

12,3 g (0,047 mol) N-(2,6-Dimethylphenyl)-alaninmethylester werden in 70 ml trockenem Toluol gelöst. Man gibt 5,88 g (0,07 mol) Natriumhydrogencarbonat hinzu und tropft bei 0°C unter heftigem Rühren 5,07 g (0,056 mol) Acrylsäurechlorid gelöst in 20 ml trockenem Toluol zu. Es wird 4 Stunden bei 25°C gerührt, abgesaugt und der Rückstand mit Toluol ausgewaschen. Die vereinigten Filtrate werden im Vakuum vom Lösungsmittel befreit und bei 50°C und 0,1 mbar 4 Stunden getrocknet. Man erhält 13,4 g (98 % d.Th.) der obengenannten Verbindung als gelbliches Öl.

b) Herstellung von N-(3-Methyl-2-isoxazolin-5-yl-carbonyl)-N-(2,6-dimethylphenyl)-2-aminopropionsäuremethylester

115,9 g (0,44 mol) N-Acryloyl-N-(2,6-dimethylphenyl)--2-aminopropionsäuremethylester werden in 700 ml Methylenchlorid gelöst und mit 41,6 g (0,48 mol) 70 %iger (Gew.%) wäßriger Acetaldoximlösung versetzt. Bei 15 bis 20°C tropft man unter heftigem Rühren langsam 395 g (0,53 mol) 10 %ige wäßrige Natriumhypochloritlösung zu und läßt 12 Stunden rühren. Dann werden die Phasen getrennt; die organische Phase wird fünfmal mit je 400 ml Wasser nachgewaschen, über Natriumsulfat getrocknet und eingedampft. Man erhält die obengenannte Verbindung als Diastereomerengemisch. Ausbeute: 130 g (92 % d.Th.), Schmelzpunkt: 91 bis 94°C (Verbindung Nr. 1).

Beispiel 2

Herstellung von

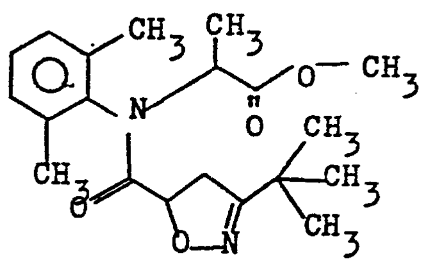

N-(3-tert.Butyl-2-isoxazolin-5-yl-carbonyl)-N-(2,6-dimethylphenyl)-2-aminopropionsäuremethylester

13,05 g (0,05 mol) N-Acryloyl-N-(2,6-dimethylphenyl)-2-aminopropionsäuremethylester werden in 75 ml Methylenchlorid gelöst und mit 5,05 g (0,05 mol) Pivalaldoxim versetzt. Unter kräftigem Rühren tropft man bei 15 bis 20°C 67 g (0,09 mol) 10 %ige Natriumhypochloritlösung zu und rührt noch 24 Stunden. Dann verdünnt man mit 100 ml Methylenchlorid, trennt die Wasserphase ab, extrahiert diese noch zweimal mit Methylenchlorid und befreit die vereinigten organischen Phasen nach Trocknen über $Na_2SO_4$ im Vakuum vom Lösungsmittel. Dabei kristallisieren 12,3 g (81 % d.Th.) der obengenannten Verbindung als Diastereomerengemisch mit Schmelzpunkt 56°C aus (Verbindung Nr. 2).

Beispiel 3

Herstellung von

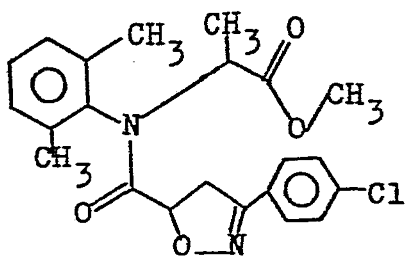

N-(3-(4-Chlorphenyl)-2-isoxazolin-5-yl-carbonyl)-N-(2,6-dimethylphenyl)-2-aminopropionsäuremethylester

13,05 g (0,05 mol) N-Acryloyl-N-(2,6-dimethylphenyl)-2-aminopropionsäuremethylester und 9,5 g (0,05 mol) 4-Chlor-benzhydroxamsäurechlorid löst man in 60 ml trockenem Toluol auf und tropft bei 20°C 7,66 ml (0,055 mol) Triethylamin gelöst in 30 ml trockenem Toluol zu. Nach 48stündigem Rühren werden weitere 7,66 ml (0,055 mol) Triethylamin in 30 ml Toluol zugetropft. Man rührt nochmals 12 Stunden, saugt vom ausgefallenen Feststoff ab und trocknet das Filtrat im Vakuum. Der harzige Rückstand wird dreimal mit je 50 ml Petrolether gründlich durchgerührt und jeweils dekantiert. Durch vierstündiges Trocknen des Harzes bei 50°C und 0,1 mbar erhält man 6,5 g (31 % d.Th.) der obengenannten Verbindung in Form zerfließlicher Kristalle (Verbindung Nr. 3) als Diastereomerengemisch.

$^1$H-NMR ($CDCl_3$): 1,1 ppm (CH-$\underline{CH_3}$), 2,2 und 2,5 ppm ($C_6H_5$-$\underline{CH_3}$), 3,1 ppm ($\underline{CH_2}$-C=N), 3,8 ppm (C-$\underline{CH_3}$), 4,5 ppm (2x -$\underline{CH}$-C=O), 7,0 bis 7,6 ppm (aromat. H)

Entsprechend wurden folgende 2-Isoxazolin-5-carbonsäureamide hergestellt:

| Nr. | Ar | $R^1$ | $R^2$ | $R^7$ | $R^8$ | IR-Spektrum (Film); Schmp. °C |
|---|---|---|---|---|---|---|
| 4 | 2,6-Dimethyl-phenyl | 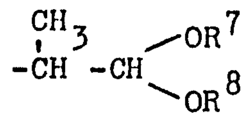 | Methyl | Methyl | Methyl | 2951,1663,1470,1457,1437, 1386,1326,1278,1258,1113, 1095,1071 $cm^{-1}$ |
| 5 | 1-(2-Methyl-naphthyl) | 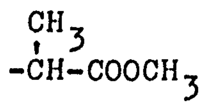 | Methyl | - | - | 2952,1748,1669,1456,1434, 1402,1385,1326,1279,1248, 1202,1180,1163,1141,821, 790 $cm^{-1}$ |
| 6 | 2,6-Dimethyl-phenyl | 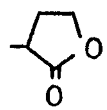 | Methyl | - | - | Schmp. 60-63°C |
| 7 | 1-(2-Methyl-naphthyl) | " | Methyl | - | - | Schmp. 72-78°C |
| 8 | 2,6-Dimethyl-phenyl | 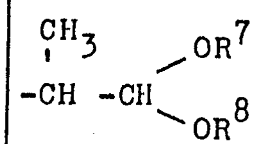 | Methyl | $-C_2H_4-$ | | 2892,1663,1434,1396,1385, 1327,1258,1233,1111,1089, 1058 $cm^{-1}$ |
| 9 | " | " | 3,4-Dichlor-phenyl | Methyl | Methyl | Schmp. 122°C |
| 10 | " | " | tert.Butyl | Methyl | Methyl | 2966,1664,1470,1389,1278, 1260,1113,1072,875 $cm^{-1}$ |

Die neuen Verbindungen sind geeignet zur Bekämpfung von Pilzen, z.B. von

Phytophthora infestans an Tomaten und Kartoffeln,
Phytophthora parasitica an Erdbeeren,
Phytophthora cactorum an Äpfeln,
Pseudoperonospora cubensis an Gurken,
Pseudoporonospora humuli an Hopfen,
Peronospora destructor an Zwiebeln,
Peronospora sparsa an Rosen,
Peronospora tabacina an Tabak,
Plasmopara viticola an Reben,
Plasmopara halstedii an Sonnenblumen,
Pythium ultimum an Erbsenkeimlingen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wassser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol,

Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch in Materialschutz eingesetzt werden. DBei der Anwendung der Wirkstoffe im Materialschutz, z.B. als Fungizide für Anstrichfarben und Weich-Polyvinylchlorid, betragen die Aufwandmengen 0,05 bis 5 % (Gew.%) Wirkstoff, bezogen auf das Gesamtgewicht der zu konservierenden Farben bzw. des mikrozid auszurüstenden Polyvinylchlorids. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. 10 Gewichtsteile der Verbindung des Beispiels 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht. Durch Eingießen der Mischung in Wasser erhält man eine wäßrige Dispersion.

II. 20 Gewichtsteile der Verbindung des Beispiels 3 werden in einer Mischung gelöst, die aus 60 Gewichtsteilen Cyclohexanon, 30 Gewichtsteilen Isobutanol, 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

III. 20 Gewichtsteile der Verbindung Nr. 9 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 80 Gewichtsteile der Verbindung des Beispiels 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle

vermahlen. Durch feines Verteilen der Mischung in Wasser erhält man eine Spritzbrühe.

V. 3 Gewichtsteile der Verbindung Nr. 4 werden mit 97 Gewichtsteilen feinteiligem Kaolin innig vermischt. Man erhält auf diese Weise ein Stäubemittel, das 3 Gew.% des Wirkstoffs enthält.

VI. 30 Gewichtsteile der Verbindung des Beispiels 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VII. 40 Gewichtsteile der Verbindung Nr. 5 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion.

VIII. 20 Teile der Verbindung Nr. 6 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Mittel können auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den neuen Verbindungen kombiniert werden können, sind beispielsweise:

Schwefel,
Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat und
Zinkethylenbisdithiocarbamat,
Tetramethylthiuramdisulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid;

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Substanzen, wie
N-(1,1,2,2-Tetrachlorethylthio)-tetrahydrophthalimid,
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diethyl-phthalimidophosphonothioat,
5-Amino-1-(bis-(dimethylamino)-phosphinyl)-3-phenyl-1,2,4-
-triazol,
5-Ethoxy-3-trichlormethyl-1,2,4-thiadiazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethyl-
ester,
2-Methoxycarbonylamino-benzimidazol,
2-Rhodanmethylthio-benzthiazol,
4-(2-Chlorphenylhydrazono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-(2))-benzimidazol,
Piperazin-1,4-diyl-bis-(1-(2,2,2-trichlor-ethyl)-formamid),
2-(Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,

sowie verschiedene Fungizide, wie
Dodecylguanidinacetat,
3-(3-(3,5-dimethyl-2-oxycyclohexyl)-2-hydroxyethyl)-glutar-
amid,

Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Cyan-N-(ethylaminocarbonyl)-2-(methoxyimino)-acetamid,
2-Methyl-benzoesäure-anilid,
2-Iod-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze.

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
1-(1',2',4'-Triazolyl-1')-1-(4'-chlorphenoxy)-3,3-dimethylbutan-2-on,
1-(1',2',4'-Triazolyl-1')-1-(4'-chlorphenoxy)-3,3-dimethylbutan-2-ol,
N-(2,6-Dimethylphenyl)-N-chloracetyl-D,L-2-aminobutyrolacton,
N-(n-Propyl)-N-(2,4,6-trichlorphenoxyethyl)-N'-imidazolylharnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin.

Für die folgenden Versuche wurden als bekannte fungizide Wirkstoffe die Verbindungen

N-Trichlormethylthio-phthalimid (A)
N-Trichlormethylthio-tetrahydrophthalimid (B)
Zink-ethylen-1,2-bisdithio-carbamat (C) verwendet.

Versuch 1

Wirksamkeit gegen Plasmopara viticola

Blätter von Topfreben der Sorte "Müller-Thurgau" werden mit wäßriger Spritzbrühe, die 80 % Wirkstoff und 20 % Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24°C und anschließend 8 Tage in einem Gewächshaus mit Temperaturen zwischen 20 und 30°C aufgestellt. Nach dieser Zeit werden die Pflanzen zur Beschleunigung des Sporangienträgerausbruchs abermals für 16 Stunden in der feuchten Kammer aufgestellt. Dann erfolgt die Beurteilung des Ausmaßes des Pilzausbruchs auf den Blattunterseiten.

Das Ergebnis des Versuches zeigt, daß beispielsweise die Wirkstoffe 1 und 2 bei Anwendung als 0,025%ige Spritzbrühe eine bessere fungizide Wirkung zeigen (beispielsweise 97 %) als der bekannte Wirkstoff A (beispielsweise 90 %).

Versuch 2

Wirksamkeit gegen Phytophthora infestans an Tomaten

Blätter von Topfpflanzen der Sorte "Große Fleischtomate" werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18°C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Wirkstoffe 1, 2, 3, 5, 6, 7 bei Anwendung als 0,025%ige Spritzbrühe eine bessere fungizide Wirkung zeigen (beispielsweise 97 %) als die bekannten Wirkstoffe B und C (beispielsweise 70 %).

Patentansprüche

1. 2-Isoxazolincarbonsäureamid der allgemeinen Formel I

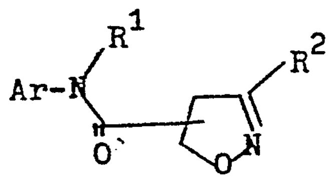 I

worin Ar

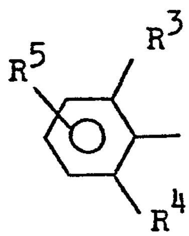 oder 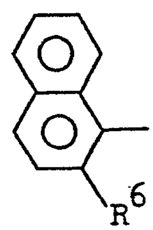 bedeutet,

und
$R^3$ $C_1$- bis $C_3$-Alkyl oder Halogen, $R^4$ $C_1$- bis $C_3$-Alkyl, $R^5$ Wasserstoff, $C_1$- bis $C_3$-Alkyl oder $C_1$- bis $C_3$--Alkoxy, $R^6$ Methyl und

$R^1$ 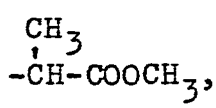, 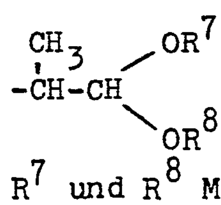 oder

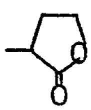 bedeutet, wobei $R^7$ und $R^8$ Methyl oder Ethyl bedeuten oder $R^7$ und $R^8$ zusammen eine $C_2$- bis $C_3$-Alkylengruppe bedeuten, die zusammen mit dem

Rest 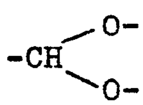 einen gegebenenfalls durch $C_1$- bis $C_3$-Alkyl substituierten Dioxolan- oder 1,3-Dioxanring bilden, und $R^2$ Wasserstoff, $C_1$- bis $C_4$-Alkyl oder einen gegebenenfalls durch Halogen substituierten Phenylrest bedeutet.

2. 2-Isoxazolin-5-carbonsäureamid gemäß Anspruch 1, dadurch gekennzeichnet, daß $R^2$ Methyl, $R^3$ und $R^4$ Methyl oder Ethyl und $R^5$ Wasserstoff bedeuten.

3. Verfahren zur Herstellung von Verbindungen gemäß Anspruch 1, dadurch gekennzeichnet, daß man ein aromatisches Amin der Formel II

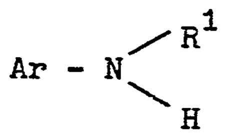 II

worin Ar und $R^1$ die in Anspruch 1 angegebene Bedeutungen haben, mit Acrylsäurechlorid gegebenenfalls in Gegenwart eines Lösungsmittels, ggf. unter Zusatz einer anorganischen oder organischen Base und ggf. unter Zusatz eines Reaktionsbeschleunigers bei Temperaturen zwischen -10 und +100°C umsetzt und an das so erzeugte Acrylsäureamid der allgemeinen Formel III

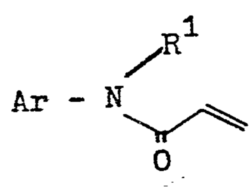 III

ein in situ erzeugtes Nitriloxid der allgemeinen Formel IV

$$R^2-C\equiv \overset{\oplus}{N}-O^{\ominus} \quad IV$$

in Gegenwart eines Lösungsmittels bei Temperaturen zwischen -10 und +60°C cycloaddiert.

4. Fungizid, enthaltend eine Verbindung gemäß Anspruch 1.

5. Fungizid, enthaltend eine Verbindung gemäß Anspruch 2.

6. Fungizid, enthaltend ein 2-Isoxazolin-carbonsäureamid gemäß Anspruch 1 und einen festen oder flüssigen Trägerstoff.

7. Verfahren zur Bekämpfung von Pilzen, dadurch gekennzeichnet, daß man ein 2-Isoxazolincarbonsäureamid gemäß Anspruch 1 auf die Pilze oder auf durch Pilzbefall bedrohte Flächen, Pflanzen oder Saatgüter einwirken läßt.

Patentansprüche (für Österreich)

1. Fungizid, enthaltend ein 2-Isoxazolincarbonsäureamid der allgemeinen Formel I

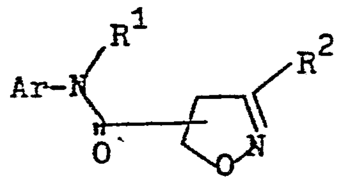

I

worin Ar

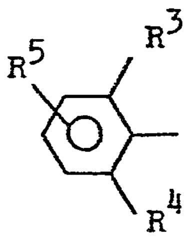

oder

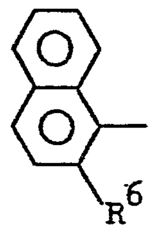

bedeutet,

und

$R^3$ $C_1$- bis $C_3$-Alkyl oder Halogen, $R^4$ $C_1$- bis $C_3$-Alkyl, $R^5$ Wasserstoff, $C_1$- bis $C_3$-Alkyl oder $C_1$- bis $C_3$--Alkoxy, $R^6$ Methyl und

$R^1$ 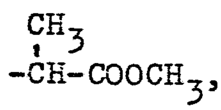, 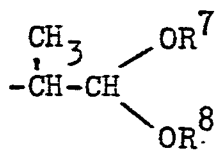 oder

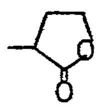 bedeutet, wobei $R^7$ und $R^8$ Methyl oder Ethyl bedeuten oder $R^7$ und $R^8$ zusammen eine $C_2$- bis $C_3$-Alkylengruppe bedeuten, die zusammen mit dem

Rest 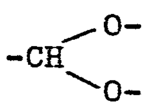 einen gegebenenfalls durch $C_1$- bis

$C_3$-Alkyl substituierten Dioxolan- oder 1,3-Dioxanring bilden, und $R^2$ Wasserstoff, $C_1$- bis $C_4$-Alkyl oder einen gegebenenfalls durch Halogen substituierten Phenylrest bedeutet.

2. Fungizid gemäß Anspruch 1, <u>dadurch gekennzeichnet</u>, daß $R^2$ Methyl, $R^3$ und $R^4$ Methyl oder Ethyl und $R^5$ Wasserstoff bedeuten.

3. Verfahren zur Herstellung von Verbindungen gemäß Anspruch 1, <u>dadurch gekennzeichnet</u>, daß man ein aromatisches Amin der Formel II

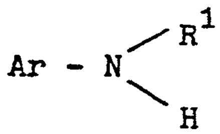 II

worin Ar und $R^1$ die in Anspruch 1 angegebene Bedeutungen haben, mit Acrylsäurechlorid gegebenenfalls in Gegenwart eines Lösungsmittels, ggf. unter Zusatz einer anorganischen oder organischen Base und ggf. unter Zusatz eines Reaktionsbeschleunigers bei Temperaturen zwischen -10 und +100°C umsetzt und an das so erzeugte Acrylsäureamid der allgemeinen Formel III

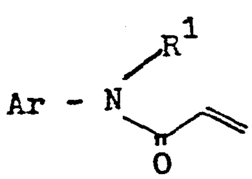 III

ein in situ erzeugtes Nitriloxid der allgemeinen Formel IV

$$R^2-C\equiv\overset{\oplus}{N}-O^{\ominus} \qquad IV$$

in Gegenwart eines Lösungsmittels bei Temperaturen zwischen -10 und +60°C cycloaddiert.

4. Fungizid, enthaltend ein 2-Isoxazolin-carbonsäureamid gemäß Anspruch 1 und einen festen oder flüssigen Trägerstoff.

5. Verfahren zur Bekämpfung von Pilzen, dadurch gekennzeichnet, daß man ein 2-Isoxazolincarbonsäureamid gemäß Anspruch 1 auf die Pilze oder auf durch Pilzbefall bedrohte Flächen, Pflanzen oder Saatgüter einwirken läßt.

Europäisches Patentamt

# EUROPÄISCHER RECHERCHENBERICHT

0078999

Nummer der Anmeldung

EP 82 10 9970

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | EP-A-0 037 927 (BASF)<br>* Ansprüche * | 1,2,4-7 | C 07 D 261/04<br>C 07 D 413/12<br>A 01 N 43/80 |
| Y | EP-A-0 026 873 (BASF)<br>* Ansprüche * | 1,2,4-7 | |
| Y | EP-A-0 029 996 (BASF)<br>* Ansprüche 1-3,5,6 * | 1,2,4-7 | |

RECHERCHIERTE SACHGEBIETE (Int. Cl. 3)

C 07 D 261/00
C 07 D 413/00

Der vorliegende Recherchenbericht wurde für alle Patentansprüche erstellt.

| Recherchenort | Abschlußdatum der Recherche | Prüfer |
|---|---|---|
| DEN HAAG | 03-02-1983 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82